# EUROPEAN PATENT APPLICATION

(11) **EP 0 733 383 A2**
(43) Date of publication of application: **25.09.1996**
(21) Application number: 96109224.4
(22) Date of filing: 02.11.1989
(51) Int. Cl.: A61N 1/05

(54) **Apparatus for removing an elongated structure implanted in biological tissue**

(30) Priority: 09.11.1988 US 269771; 17.01.1989 US 298100; 03.05.1989 US 347217; 09.06.1989 US 363960
(62) Divisional of application: 89311373.8
(71) Applicant: MED INSTITUTE, INC., West Lafayette Indiana 47906 (US); COOK PACEMAKER CORPORATION, Leechburg Pennsylvania 15656 (US)
(72) Inventor: Goode, Louis, Evans City, Pennsylvania 16033 (US); Fearnot, Neal Edward, West Lafayette, Indiana 47906 (US); Shipko, Frederick John, Spring Church, Pennsylvania 15686 (US)
(74) Representative: Johnston, Kenneth Graham

(57) **Abstract**

Heart lead removal apparatus and method are disclosed for removing a heart lead from the wall of a heart through a blood vessel leading to the heart. The apparatus comprises a flexible stylet with an expandable member inserted within a coiled structure of the heart lead. When expanded the member grasps the distal end of the stylet and enables it to be extracted without rupture of the stylet.

## Description

This invention relates to elongated structures, such as a catheter implanted in biological tissue, ducts etc. or an electrical pacemaker lead implanted in the heart and, particularly, to apparatus for removing such elongated structures.

A heart pacemaker comprising an electrical wire coil or lead usually is surgically implanted through a vein such as the right subclavian vein leading to a cavity such as the superior vena cava, and to the right ventricle of the heart. A typical lead includes one or more helical wire coils having a hollow inner passageway that extends the entire length of the wire coil. The coiled structures are positioned in the lead either coaxially or laterally, and are insulated from body fluids by insulating material (silicon or polyurethane). However, one problem is that, over the years, fibrotic tissue commonly encapsulates the pacemaker lead especially in areas where there is low velocity blood flow. Separation of the lead from the vein can cause severe damage to the vein.

Should a heart lead need to be replaced at any time, it is undesirable to leave the existing pacemaker lead in place.

If removal of a lead from the heart has been required for any reason, it has often involved open heart surgery with accompanying complications, risks, and significant cost.

Prior art apparatus or tools for removing pacemaker leads have not proved satisfactory. Once locked in position they cannot be disengaged from the locking relationship with the pacemaker leads. As a result, the tool and lead must be surgically removed. Furthermore, certain tools can easily puncture a blood vessel or, even worse, a heart cavity wall. It has been known for a coiled structure to unwind and damage the heart and surrounding blood vessels. Pacemaker leads can include tines or a corkscrew at the tip or a conically shaped tip for securing the distal end of the pacemaker lead to a heart cavity wall. For fibrotic tissue that has encapsulated the tip, unaided manual removal of the heart lead from the heart cavity wall may cause an inward extension or inversion of the wall, or even worse, permanent damage to the heart such as tearing a hole in the heart cavity wall.

U.S. Patent 4,574,800 discloses apparatus for removing implanted cardiac pace making leads, said apparatus being as defined in the preamble of claim 1.

U.S. Patent 4,471,777 also discloses apparatus for removing similar types of leads. The apparatus uses grasping claws for securing the proximal ends of such claws.

The present invention provides apparatus as defined in claim 1 for removing an elongated structure implanted in biological tissue.

### Brief Description of the Drawing

Figs. 1 to 5, Figs. 7 to 14, and Figs. 17 to 24, illustrate sections through apparatus useful for providing an understanding of the invention; they are not embodiments of the present invention;
Fig. 6 illustrates an embodiment of the present invention; and
Figs. 15 and 16 illustrate another embodiment of the present invention.

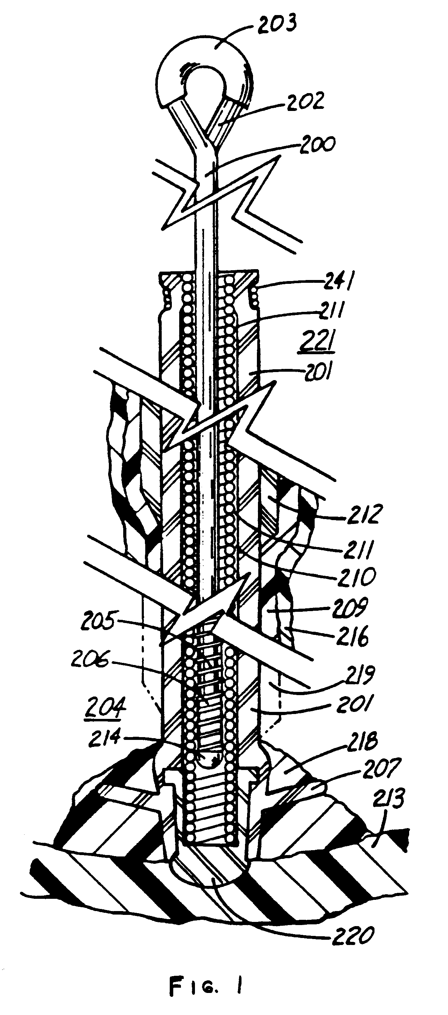

Fig. 1 shows an electrical heart pacemaker lead 204 secured to the apex 213 of the right ventricle with a plurality of tines 207, which in time become securely attached to the ventricle wall by endothelial and/or fibrotic tissue forming around the heart lead tip with electrode 220 in contact with the lead and the ventricle wall 213. Some ventricles are relatively smooth on the inside, but most have trabeculae amongst which the tines are secured into position. External to the right subclavian vein, the proximal end 221 of the lead can be grasped by a lackable clamp mechanism which will be described hereinafter.

A flexible stylet member such as wire 200 of the present lead removal apparatus is inserted in the longitudinal passageway 210 of the heart lead coiled structure 211 for controlling and, in particular, limiting the movement of heart lead 204 including coiled structure 211. Heart lead 204 also includes insulating material 201, such as silicon or polyurethane, formed into a hollow tube that surrounds the coiled structure and prevents fluids from making contact with or entering the coiled structure. Attached to the flexible stylet wire is an expandable wire coil 205 consisting of approximately 25 turns of wire with spacing between the turns. Five to seven wraps of the wire coil are attached preferably close to the distal end of the stylet wire using, for example, solder 206. The remaining wraps of the wire coil remain free for engaging the coiled structure when the proximal end of the stylet wire is rotated in a direction to unwind and expand the turns of the wire coil and engage the coiled structure of the heart lead. A bead 214 of high temperature silver solder is applied to the distal end of the stylet wire to prevent the distal end thereof from pulling through the wire coil during separation and removal of the heart lead. Positioned about the proximal end of the stylet wire is control mechanism 202 for rotating the stylet wire in either a clockwise or counterclockwise direction or for moving the wire in a longitudinal direction into or out of the passageway. In this embodiment, control mechanism 202 is a loop of wire formed from the stylet wire of which the physician may grasp or insert his finger.

The choice of the stylet wire and wire coil varies with the internal diameter of the coiled structure which varies from 0.04cms. (0.016") to about 0.071cms (0.028") for most heart leads. The diameter of the stylet wire would then range from 0.023cms (0.009") to 0.038cms (0.015"), with the coil wire ranging in diameter from 0.008cms (0.003") to 0.0152cms (0.006"). The use of stainless steel wire is preferable. The stylet wire should be hardened wire, but ductable wire may be used for the coil wire.

Before the stylet wire is inserted into passageway 210 of the lead, the inside diameter of the coiled structure and the outside diameter of the insulating material are determined. A lockable clamp is first applied to the proximal end 221 of the lead, the latter being in channels in pliable opposing semicircular jaws of the clamp.

When proximal end 221 of lead 204 is inserted and grasped in the hollow passageway, the insulating material 201 is compressed onto coiled structure 211, thus limiting the movement of the structure within the insulating material. When the physician cuts the lead for access to the passageway of the lead, the compressed insulating material prevents the coiled structure from retracting into the passageway of the lead.

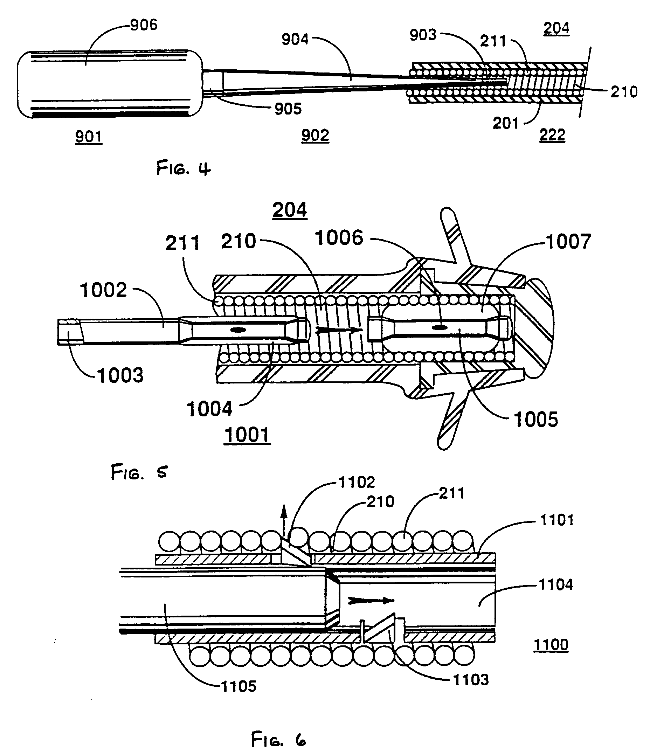

With the lockable clamp applied to the proximal end 221 of the pacemaker lead, the electrical connector (not shown) is severed from the pacemaker lead 204. The severance could deform the passageway 210 thereby presenting a false indication of the actual diameter thereof. As a consequence, the physician inserts the pointed end 903 of a tapered rod 904 of an expansion device 901 into the proximal end of hollow passageway 210 to expand coiled structure 211, as shown in FIG.4.

A physician can examine the passageway by inserting a wire guide therein and sensing for any blockages. The physician can extend the guide into the lead passageway and rotate the guide back and forth to clear any blockages caused by tissue or occluding material. The guide can also be used to determine or size the inside diameter of a second coiled structure that may be coaxially positioned inside coiled structure 211. When utilized as a control mechanism for stylet wire 200, the guide may also include appendages for rotating and counting the number of times the stylet wire is rotated. Having determined the lead passageway with the wire guide, several other similar guides can be individually inserted in the passageway to determine the actual inside diameter at the proximal end. The guide can also be utilized to determine if coiled structure 211 has been deformed or damaged and to determine the smallest diameter of the coiled structure and passageway.

The outer diameter of the coil wire and stylet wire have been selected to form a combined overall diameter which approximates the inner diameter of the longitudinal passageway of the heart lead coiled structure within a predetermined tolerance such as 0.00254 to 0.005cms. (one or two thousandths of an inch). Stylet wire 200 is fed through the entire length of the passageway to the distal end of the coiled structure. When fully inserted into the heart lead, the distal ends of the stylet wire and coiled structure should be in close proximity. It is not necessary, but probably more advantageous, that the stylet wire be attached to the distal end of the heart lead. For separating the heart lead from adjacent tissue, the stylet wire may be secured anywhere along the passageway of the coiled structure past the restricting tissue. To secure the stylet wire to coiled structure 211, looped end 202 of the stylet wire is operated in a circular direction to unwind and expand wire coil 205. As a result, the turns of the wire coil and coiled structure engage and intermesh, thereby firmly securing the stylet wire to the heart lead. This prevents any extension or stretching of the heart lead and also controls and limits the movement of the lead when separator tube 212 is moved along the length of coiled structure 211 and insulating material 201 of the heart lead.

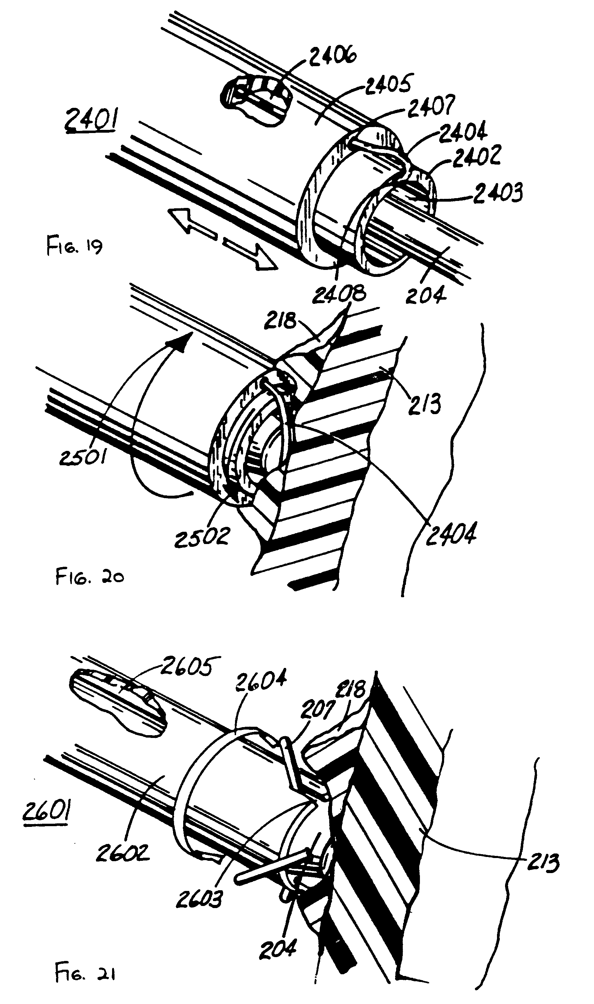

FIG. 18 illustrates another form of stylet, including a stylet wire 2302 with a conically-shaped silver solder tip 2303 that is positioned at the distal end thereof. Closely wrapped wire coil 2304, similar to wire coil 205, is attached at the distal end of the stylet wire using silver solder 2305 as previously described. The proximal end of the wire coil is pulled to unwrap several turns to form a catch wire 2306 to extend in a radial direction from the wire coil and stylet wire. Catch wire 2306 catches on or engages the coiled structure of the pacemaker lead to engage wire coil 2304 with the coiled structure of the pacemaker lead. In addition, the wire coil may be rotated in the opposite direction to release the stylet wire from the coiled structure if desired.

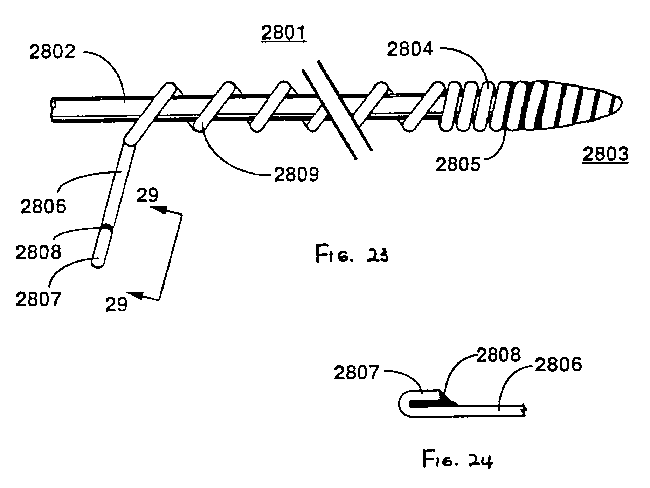

FIG.23 illustrates a further form of stylet with a catch wire 2806 similar to that in the stylet of FIG.18. At the distal end 2803, closely-spaced turns 2804 are attached to stylet wire 2802 using silver solder 2805. The distal end 2803 is shaped for easy insertion into passageway 210. The open-spaced turns 2809 engage with the coiled structure of the pacemaker lead. To more readily engage the coiled structure, the proximal end 2807 of the catch wire is folded back on itself and attached thereto with silver solder 2808. A side view of the proximal end of catch wire 2806 taken along the line 29-29 is depicted in FIG.24. The proximal end 2807 is folded back and attached to catch wire 2806 so as not to extend beyond the thickness of the wire as shown in FIG.23. This is to prevent the catch wire from engaging the coiled structure of the pacemaker lead or binding between the stylet wire and coiled structure when the removal apparatus is being inserted in the passageway of the coiled structure. Experiments in the laboratory and in actual pacemaker lead removals indicate that the length of catch wire 2806 should be preferably 0.635cms (one-quarter of an inch) in length. The folded-back segment end 2807 should preferably be 0.159cms. (one-sixteenth of an inch) in length. As a result, only a few counterclockwise turns of the stylet wire in laboratory tests were found necessary to engage the coiled structure of the pacemaker lead. Previously, ten to twenty turns were required to engage the coiled structure when the catch wire did not include the folded-back proximal end.

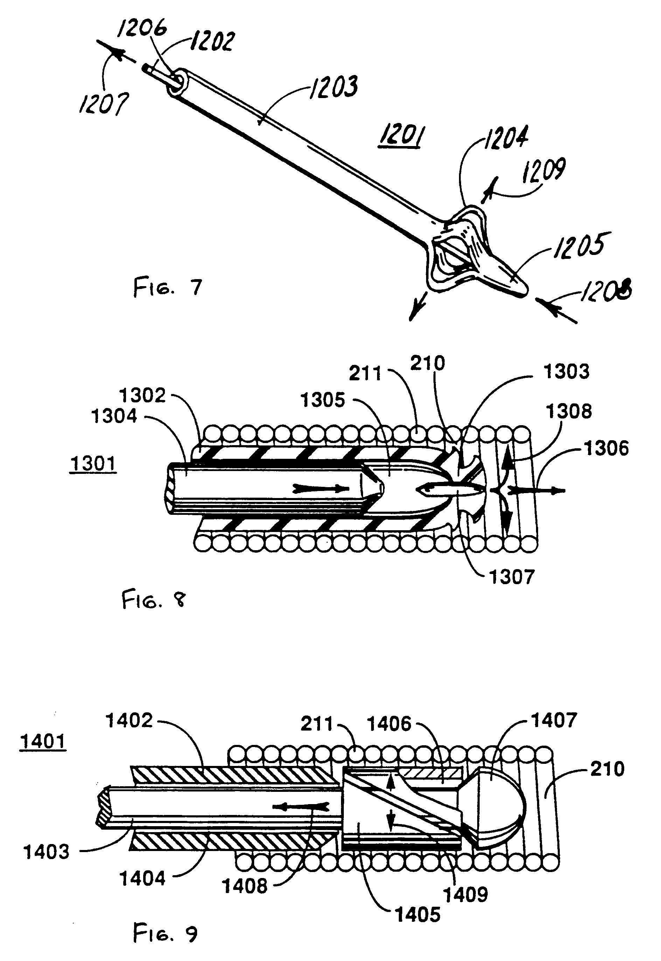

FIGS.5-16 illustrate further alternative stylets. However, the control unit in each of these alternative embodiments commonly, but not in all cases, includes a longitudinal passageway for operating the expandable unit to the expanded position for securing the control unit to the elongated structure.

The stylet or control unit of FIG.5 removal apparatus includes a flexible tube 1002 having a passageway 1003 formed longitudinally therein. Expandable balloon 1004 is positioned and attached about or near the distal end of the control tube and is expanded at 1007 by compressed gas or liquid via tube 1003. The distal end of the control tube is also recessed to attach to the balloon in a well-known manner at the ends of radial recess 1005. The recess also provides a volume in which the collapsed balloon is stored. The recess also includes one or more side ports 1006 leading from passageway 1003 to the balloon.

The embodiment of FIG.6 comprises a stylet or control tube 1101 for insertion into passageway 210. An expandable unit comprising a plurality of radial projections 1102 and 1103 that have a free end are radially formed in or near the distal end of the control tube. The free end of each radial projection is twisted and bent in an inward direction into passageway 1104 of the control tube. As formed, these projections allow a control tube to be easily inserted into passageway 210. When control tube 1101 is positioned at the distal end of the coiled structure, actuator rod 1105 is inserted in passageway 1104 of the control tube. When inserted, the actuator rod engages the radial projections and forces them into an expanded position extending radially from the surface of the control tube into the coiled structure of the pacemaker lead. When in the expanded position, these radial projections secure the control tube to the coiled structure, thereby controlling movement of the coiled structure during removal from the tissue. In order to remove the control tube from passageway 210, if desired, the rod 1105 is removed.

The stylet embodiment of FIG.7 includes an actuator rod 1202, a control tube 1203 for insertion into passageway 210. Longitudinal strips 1204 are expanded by pulling (1207) the actuator rod 1202 attached to the distal end 1205 of tube 1203. The expanding strips 1204 engage the coiled structure and secure the control tube to the coiled structure of the pacemaker lead. Expanded strips 1204 can be made to contract by pushing forward rod 1202 and if desired the tube 1203 can then be removed from passageway 210.

Removal apparatus 1301 or stylet of the FIG.8 embodiment includes a control tube 1302 having a distal end with a spiral or helical ridge 1303 formed therein. Alternatively, a number of barbs are formed in the contoured distal end of control tube 1302. The distal end includes a plurality of slits 1307 or an opening thereat for expanding the ridge or barbs into the coiled structure. Actuator rod 1304 is inserted into passageway 1305 to engage the distal end. When engaged, actuator rod expands the ridge or barbs in a radial direction, as shown by arrows 1308, to engage the coiled structure 211. Disengagement of the ridge or barbs is achieved by removal of rod 1304.

In the FIG.9 embodiment, the removal apparatus or stylet includes control tube 1402 and actuator rod 1403 extending through tube 1402. A diagonally-slotted sleeve 1405 is positioned between the distal ends of the control tube and actuator rod. The actuator rod also extends through hollow passageway 1406 of the sleeve. Attached to the distal end of the actuator rod is bevelled tip 1407 having an outside diameter approximating the diameter of the control tube and the nominal diameter of the slotted sleeve. Similarly, the distal end of the control tube is bevelled to engage and expand the slotted sleeve. To expand the slotted sleeve, the actuator rod is pulled, as indicated by arrow 1408, to engage the sleeve against the bevelled edges of the control tube and the rod. As a result, the sleeve is expanded to a position for engaging coiled structure 211 and securing the control tube thereto. The slotted sleeve expands in a radial direction as indicated by arrows 1409. In order to contract the sleeve in the event that withdrawal of the stylet from passageway 210 is required, the rod 1403 is pushed forward to the distal end.

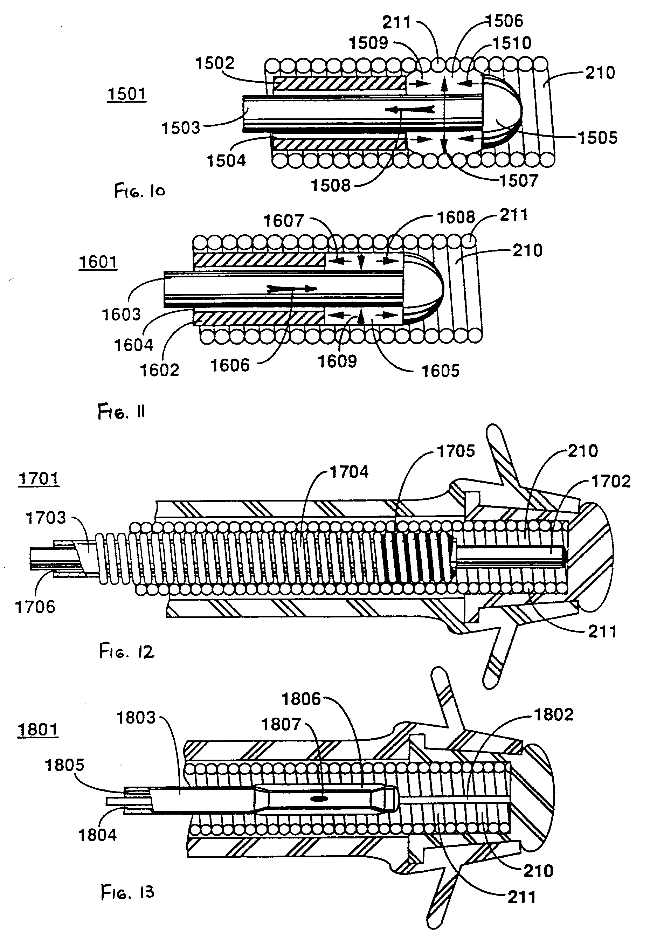

In FIG. 10, removal apparatus or stylet 1501 includes a control tube 1502 and extended therein an actuator rod 1503. The distal end of the actuator rod includes enlarged tip 1505 having a diameter approximating the diameter of the control tube. The device also includes expandable sleeve 1506 of pliable material which radially expands to engage 211 when rod 1503 is pulled in direction 1508. Removal of tube 1502 from passageway 210 can be achieved, if desired, by pushing forward rod 1503 and pulling tube 1502 in the direction 1508.

Removal apparatus 1601 or stylet depicted in FIG. 11 includes control tube 1602 having a longitudinal passageway 1604, actuator rod 1603 having an enlarged distal tip, and pliable material 1605 attached to the distal end of control tube 1602 and the distal tip. However, unlike pliable material 1506, pliable material 1605 in a relaxed condition has an outside diameter greater than the diameter of longitudinal passageway 210. Therefore, to insert the removal apparatus in the passageway or to withdraw it therefrom if required, the actuator rod is forced into passageway 1610 as indicated by arrow 1606, thereby stretching pliable material 1605 as indicated by arrows 1607 and 1608. As a result, the outside diameter of the pliable material decreases as indicated by arrows 1609 for insertion into the passageway of the elongated structure. When inserted, the actuator rod is released, and the pliable material attempts to return to its relaxed state. As a result, the pliable material engages the coiled structure and secures the device to the pacemaker lead.

In FIG. 12, removal apparatus or stylet 1701 includes wire guide 1702 that is inserted into passageway 210 to clear any blockage and establish a guide for control tube 1703. Also included is wire coil 1704 that is positioned and attached at the distal ends thereof using, for example, silver solder 1705. As previously described with respect to stylet wire 200, control tube 1703 is rotated in a direction opposite that of coiled structure 211 for engaging and expanding wire coil 1704, thereby securing the control tube to the coiled structure. The tube and coil can be removed from passageway 210 by reversing the direction of rotation.

Removal apparatus or stylet 1801 includes in FIG. 13 wire guide 1802 that is inserted into passageway 210. Control tube 1803 includes two longitudinal passageways 1804 and 1805. Passageway 1804 receives the wire guide as the control tube is inserted into the passageway of the elongated structure. Positioned at or near the distal end of the control tube is inflatable balloon 1806 with passageway 1805 leading thereto through sideport or aperture 1807. To secure the control tube to the elongated structure, a fluid is passed through passageway 1805 to inflate the balloon to an expanded position.

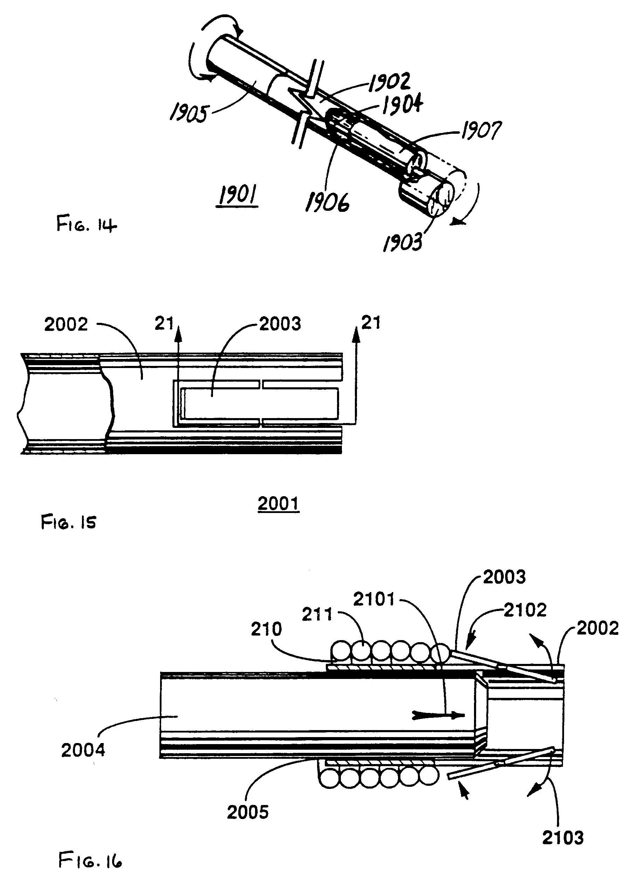

FIG. 14 illustrates removal apparatus or stylet 1901 that includes control tube 1902 and cylinder 1903. The tube includes longitudinal passageway 1904. Cylinder 1903 is positioned about the distal end of the control tube and rotated by actuator rod 1904 fixed to the cylinder, at an off-centred position, to a position off-centre of the tube for securing the control tube to the elongated structure. Actuator rod 1904 extends between the rotatable cylinder and control mechanism 1905 that is positioned at the proximal end of the control tube. Control mechanism 1905 is rotatable between two positions for rotating the actuator rod and the cylinder between expanded and retracted positions. The latter position enables removal of the stylet from the passageway 210 if required. Plug 1907 is inserted at the distal end of the tube to maintain the off-centred position of the rod in the passageway.

The stylet of FIG.15 includes a control tube 2002 with a longitudinal projection 2003 extending at the distal end thereof for securing the control tube to the coiled structure of a pacemaker lead. This arrangement is referred to as a flea-clip arrangement.

FIG.16 is a sectioned view, along the lines 21-21 in FIG.15, of the apparatus in passageway 210. As shown, a stylet wire or rod 2004 is inserted into passageway 2005 of control tube 2002 to engage and retract the extended projections into the wall of the control tube. When the apparatus is inserted to the distal end of the coiled structure, the stylet wire or rod is removed from the passageway of the control tube. As a result, the spring-like projections extend into the coiled structure and secure the control tube to the coiled structure for controlling the movement thereof. To remove the control tube from passageway 210, the rod is inserted into the control tube passageway as shown by arrow 2101 to again engage the projections. When the rod engages the projections extending into the passageway, the inward extending projections move into the wall in a direction as shown by arrows 2103, whereas the outward extending projections move into the wall in a direction as shown by arrows 2102.

After the stylet wire in FIG.1 is secured to the lead and prior to inserting separator tube 212 over the stylet wire and lead, a tie 241 of, for example, nylon cord or suture material is wrapped around proximal end 221 of the lead to secure insulating material 201 to coiled structure 211. The tie controls or limits the movement of the coiled structure within the insulating material. With the insulating material secured to the coiled structure at the proximal end, removal force is applied not only to the coiled structure, but also to the insulating material of the lead as well. This maintains the integrity of the heart lead during subsequent tissue separation from the insulating material. In those instances where the stylet wire has not been fully inserted to the distal end of the lead, the tie also prevents the coiled structure from unravelling, breaking or separating from electrode 220 or the rest of the lead.

The looped proximal end of the stylet wire can be compressed to permit separator tube 212 to be inserted thereover and over the insulating material of the heart lead. Separator tube 212 comprises a semi-rigid material, such as teflon, for sliding easily through the blood vessel and over the insulating material of the heart lead. In order to place the separator tube over the stylet, the stylet should extend at least 30.5cms. (12 inches) beyond the person's body so that the looped end can be grasped to apply tension to the stylet. The teflon separator tube 212 is 25.4 to 30.5 cms (10 to 12 inches) long, and the stylet is typically 91.4cms. (three feet) long.

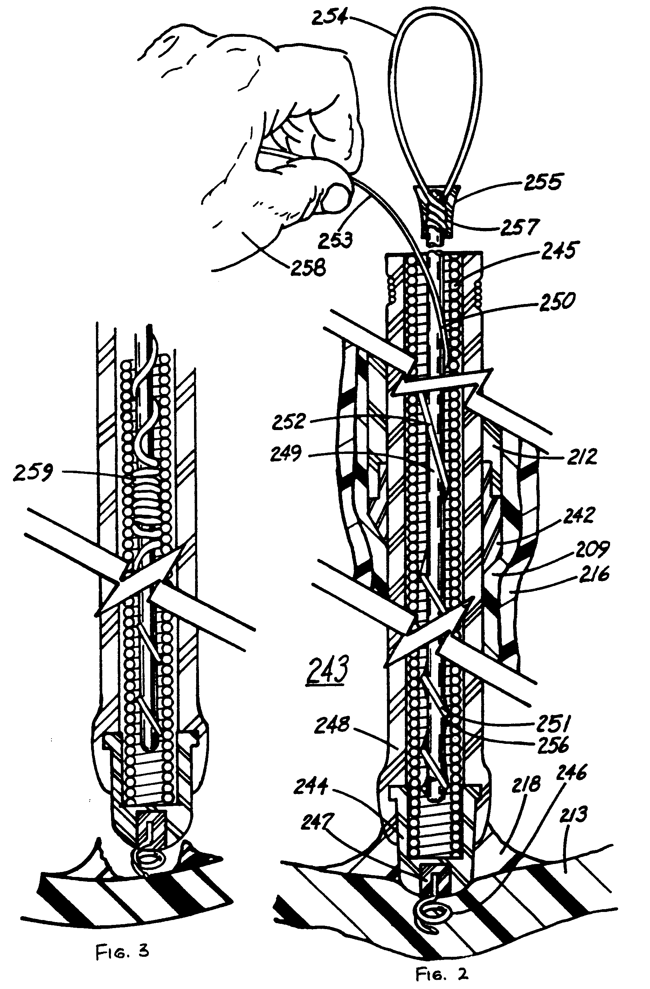

As shown, the distal end of the teflon separator tube 212 is bevelled and includes a cutting edge or edge having a number of teeth for separating heart lead insulating material 201 from encapsulating fibrotic tissue 209. As depicted in FIG.2, hollow separator tube 212 has a metal bevelled tip 242 attached to the distal end thereof with, for example, a medical grade adhesive. The metal tip provides a more durable edge for separating or cutting encapsulating fibrotic tissue from the lead.

Separator tube 212 is moved and rotated along the outer surface of insulating material 201 of the heart lead to separate the lead from the blood vessel wall. After the separator tube has been moved along the entire length of the heart lead, it will abut next to the heart cavity wall as shown by phantom lines 219. The separator tube is then rotated back and forth to dislodge and separate tines 207 and the distal end of the heart lead from fibrotic tissue 218 and heart cavity wall 213. As a result, the heart lead has now been completely separated from the blood vessel and the heart cavity wall for subsequent removal. The separator tube, the stylet wire, and the heart lead are then removed from the heart cavity and surrounding blood vessel.

Should the removal of the heart lead be prevented for whatever reason in the above embodiments, the stylet can be adjusted such as by rotating the wire in a clockwise direction to unsecure the stylet and wire coil from the heart lead coiled structure. The time for this operation is lessened by attaching a rotating mechanism such as an electrical screwdriver to the proximal end of the stylet wire.

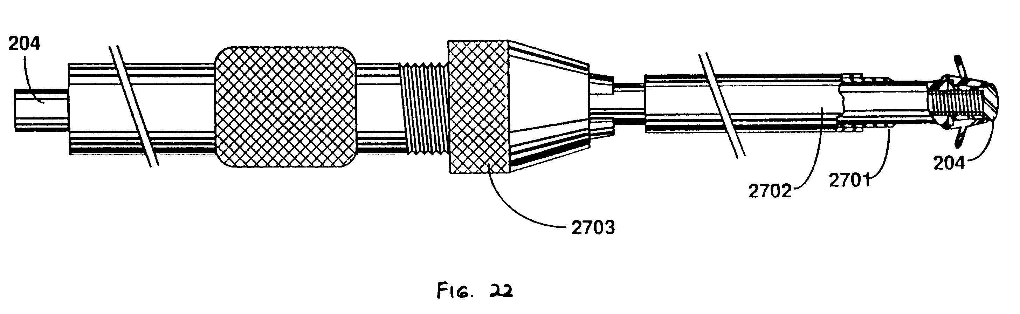

Separator apparatus or stylet of FIG.22 includes a set of separator and dilator tubes 2701 and 2702 preferably of polypropylene for insertion over pacemaker lead 204. Separator tube 2701 is similar to tube 212. Dilator tube 2702 receives separator tube 2701 and the pacemaker lead therein. The inclusion of approximately 25% of bismuth provides radio-opacity for viewing with, for example, a fluoroscope during insertion of the separator tube. When the dilator tube is inserted over the separator tube and lead, a control mechanism 2703 having a hollow passageway therein is inserted over the lead and connected to the proximal end of separator tube 2101. Control mechanism is well-known as a pinvise and is used for controlling the movement of the separator tube in both a longitudinal and rotational direction. The dilator tube and separator tube are alternatively moved along the lead to first separate the tissue from the lead and further dilate the tissue with the dilator tube. The control mechanism 2703 provides added strength and control during the movement of the separator tube. Dilator tube 2702 not only provides extra dilation of the tissue but also provides additional strength to the entire structure for separating tissue from the pacemaker lead.

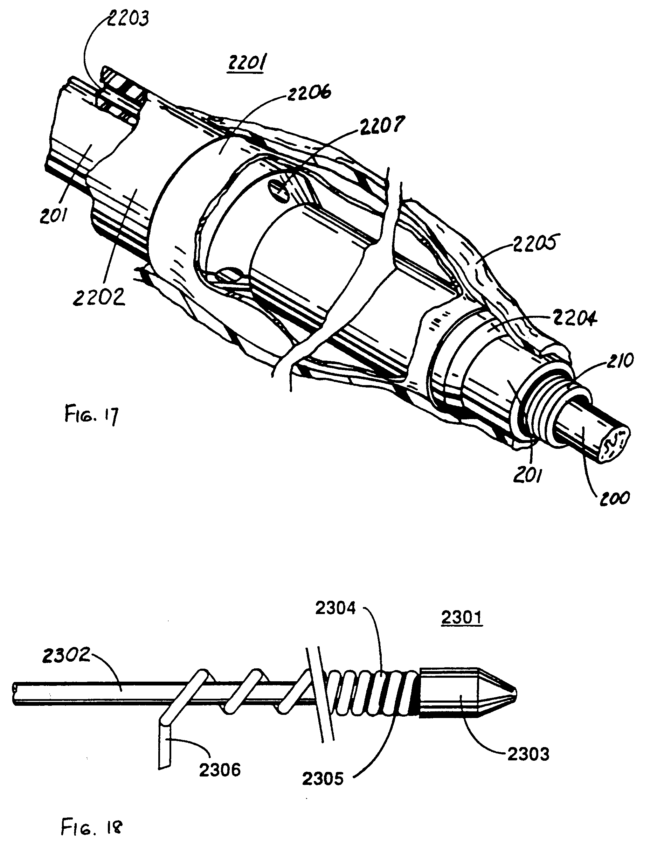

The separator apparatus 2201 of the FIG.17 embodiment includes a tube 2202 having a longitudinal passageway 2203 therein for receiving and passing over the pacemaker lead including outer insulating material 201. Distal end 2204 of the tube is bevelled to provide a wedge for separating encapsulating tissue 2205 from the pacemaker lead. Also positioned and attached in a well-known manner about the distal end of the separator tube is balloon 2206. The tube also includes a plurality of hollow passageways 2207 for supplying a compressed gas or fluid for inflating the balloon. Separator apparatus is inserted over the insulating material sheath of the pacemaker lead to engage encapsulatlng tissue 2205. The bevelled distal end provides a wedge for causing an initial separation of the tissue from the lead. Upon initial contact and separation, the balloon is inflated to provide further dilation and separation of the encapsulating tissue from the pacemaker lead. The balloon is then deflated to permit the bevelled distal end to be further moved along the pacemaker lead and engage additional encapsulating tissue. This process is continued until all of the encapsulating tissue is separated from the pacemaker lead.

Separator apparatus 2401 of FIGS. 19 and 20 separates the distal end of the structure from tissue 218. Separator apparatus includes a first tube 2402 for receiving pacemaker lead 204 and extending to the distal end thereof. Attached to the distal end of the first tube 2402 is an elongated member such as stainless steel wire 2404. The first tube wall also has a hollow channel or passageway 2408 extending longitudinally therethrough for passing the wire the entire length of the tube. Alternatively, the stainless steel wire can be affixed to the distal end. A second tube 2405 receives the first tube. In addition, the second tube similarly includes a hollow channel or passageway 2407 for extending stainless steel wire 2404 through the entire length of the tube and beyond the proximal end thereof. This permits the loose end of the wire to be controlled by the clinician to remove the distal end of the pacemaker lead from the encapsulating or affixed tissue. As shown in FIG.20, the first tube is extended to the distal end of the pacemaker lead and placed next to electrode 220. The second tube with the stainless steel wire is then also positioned next to the distal end of the pacemaker lead next to the electrode. The clinician puts tension on the stainless steel cutting wire and then rotates the second tube relative to the first causing the stainless steel wire to wipe across the face of the electrode as shown. Rotation of the two tubes are shown by arrows 2501 and 2502. This wiping motion across the pacemaker electrode literally cuts the electrode tip free from the encapsulating or affixed tissue 218. Instead of stainless steel wire, suture material is also used to perform the cutting action.

Depicted in FIG.21 is another form of separator apparatus 2601 for separating the distal end of a pacemaker lead from tissue 218. Apparatus 2601 includes tube 2602 having a longitudinal passageway 2605 for receiving pacemaker lead 204. The tube is inserted over the lead and extended to the distal end thereof. The tube includes a plurality of slots 2603 formed at the distal end for receiving pacemaker lead tines 207. When the tines are received in the slots, tube 2602 is rotated back and forth in a circular motion for dislodging and separating the tines from the encapsulating tissue 218 extending from heart wall tissue 213.

In FIG.2, electrode 244 has two cavities therein, one for receiving the coiled structure 245 and the second for receiving and securing insulated anchoring coil 246, for attaching electrode 244 to the heart tissue.

Stylet wire 249 of this lead removal apparatus and lock wire 250 attached to or near the distal end thereof have a combined diameter much less than the inside diameter of coil structure 245, which helps when the coiled structure of the lead has been deformed, unravelled, or in some way damaged. Lock wire 250 has a plurality of turns 251 closely wrapped around the distal end of the stylet wire, and attached by silver solder. Turns 252 of the lock wire are more loosely wrapped and are approximately 75 in number. The end 253 is secured whilst the stylet wire is rotated via 254 to expand lock wire turns 252 and engage the turns 245. Disengagement, if required, can be effected by rotating in the opposite direction.

When the stylet wire is secured to the pacemaker lead, loop 254 is used to extract stylet wire and pacemaker lead from the patient.

A tool such as an electrical screwdriver is attached to the control mechanism loop to rotate the stylet wire and expand the turns of the lock wire. While the stylet wire is being rotated, the physician secures the position of the proximal end 253 of the lock wire to permit lock wire turns 252 to tangle and form a bundle 259 that engages the coiled structure as depicted in FIG.8. The stylet may have to rotate 50 to 100 turns to form bundle 259 and engage coiled structure 245.

After the lock wire has secured the stylet wire to the pacemaker lead, the physician grasps control loop 254 and continues to rotate the stylet wire and pacemaker lead to dislodge anchoring coil 246 from the heart tissue. Should the blood vessels encapsulate the pacemaker lead, separator tube 212 can be employed as previously described.

The above apparatus may be utilized for removing electrical leads or other structures from body ducts and passages as well as from body tissue that has encapsulated the lead and restricted its movement.

## Claims

1. Apparatus for removing a lead with a coiled structure (211) which lead has been implanted in biological tissue, said apparatus comprising a control tube member (1101,2002) to be inserted into a longitudinal passageway (210) within the coiled structure via the proximal end of the latter, the tube member being capable of being inserted substantially along the length of the structure;
an actuator rod member (1105,2004) extending through and movable within a passageway (1104) of the control tube; and an outwardly expandable part (1102, 1103, 2003) located within the coiled structure, characterised in that the outwardly expandable part comprises a plurality of projections (1102,1103) which radially extend into the coiled structure when the members are moved relative to one another, with the free end of each projection facing the proximal end of the apparatus, whereby each free end engages a respective surface of the coiled structure, which surface faces the distal end of the apparatus.

2. Apparatus according to claim 1, wherein the said projections (2003) are hinged, and wherein the relative movement of the members causes the said free ends to radially extend into and directly engage with the coiled structure.

3. Apparatus according to claim 1, wherein withdrawal of the said lead (211) is achieved with the said members (1101,1105) fully inserted substantially adjacent to the distal end of the said lead; and with the members maintained in a fixed relationship to one another.

4. Apparatus according to claim 1,2 or 3, wherein the control tube is in engagement with the projections.

5. Apparatus according to claim 1,2,3, or 4, wherein the expandable part comprises two projections which move radially when longitudinal forces are exerted thereon, and wherein the free ends of the two projections engage diametrically opposite surfaces of the coiled structure.
